# EUROPÄISCHE PATENTANMELDUNG

(11) **EP 3 530 304 A1**
(43) Veröffentlichungstag der Anmeldung: **28.08.2019**
(21) Anmeldenummer: 18158542.3
(22) Anmeldetag: 26.02.2018
(51) Int. Cl.: A61M 5/32, A61F 9/008

(54) **KANÜLE FÜR INTRAVITREALE PUNKTION UND INJEKTION**

(71) Anmelder: Justus-Liebig-Universität Gießen, 35390 Gießen (DE)
(72) Erfinder: LYTVYNCHUK, Lyubomyr, 35394 Gießen (DE)
(74) Vertreter: Stumpf, Peter

(57) **Zusammenfassung**

Die vorliegende Erfindung betrifft eine Kanüle, welche vorteilhaft zur Punktion des Glaskörpers eines menschlichen oder tierischen Auges geeignet ist. Ebenso ist die erfindungsgemäße Vorrichtung für die intravitreale Injektion eines Medikamentes in ein menschliches oder tierisches Auge geeignet.

## Beschreibung

Die vorliegende Erfindung betrifft eine Kanüle, welche vorteilhaft zur Punktion des Glaskörpers eines menschlichen oder tierischen Auges (intravitreale Punktion) geeignet ist. Ebenso ist die erfindungsgemäße Vorrichtung für die intravitreale Injektion eines Fluids in ein menschliches oder tierisches Auge geeignet. Das Fluid kann eine Flüssigkeit oder ein Gas sein. Bei dem Fluid kann es sich um ein Fertigarzneimittel oder eine andere pharmazeutische Zubereitung oder ein pharmazeutisches Produkt handeln. Im dem Fluid können ein oder mehrere Wirkstoffe gelöst werden, es handelt sich dann um ein Medikament.

Eine Kanüle (Synonyme: Hohlnadel, Injektionsnadel, Punktionsnadel) ist eine hohle Nadel bzw. ein dünnes, zylindrisches Rohr, welche für die Punktion eines menschlichen oder tierischen Körpers geeignet ist. Eine Kanüle weist üblicherweise eine Spitze, einen Schaft und einen Ansatz auf. Kanülen werden üblicherweise aus hygienischen Gründen nach dem Gebrauch entsorgt, es handelt sich also meist um Einmal-Wegwerfprodukte. Kanülen werden üblicherweise aus Stahl hergestellt, der Kanülenansatz besteht meist aus Kunststoff. Es können aber auch andere geeignete Materialien für die Herstellung einer Kanüle verwendet werden.

Eine Punktion ist das gezielte Einstechen einer Kanüle in einen menschlichen oder tierischen Körper. Die Punktion kann an den verschiedensten Stellen des menschlichen oder tierischen Körpers durchgeführt werden. Je nachdem an welcher Stelle die Punktion mit einer Kanüle durchgeführt wird, welches Gewebe sich dort befindet und mit welcher Absicht die Punktion durchgeführt wird, werden unterschiedliche Kanülen verwendet. Die verschiedenen Kanülen unterscheiden sich unter anderem in ihrem Durchmesser, ihrer Länge, ihrer Form der Spitze und in dem Material aus dem sie bestehen. Üblicherweise sind Kanülen gerade ausgebildet, für besondere Einsatzzwecke können Kanülen auch gebogen, gekrümmt oder abgeknickt sein.

Eine Kanüle ist dadurch gekennzeichnet, dass ein Ende in der Regel als Ansatzstück für eine Spritze oder einen Schlauch ausgebildet ist (Kanülenansatz) und das andere Ende als Spitze (Kanülenspitze). Der dazwischenliegende Kanülenschaft ist üblicherweise zylindrisch ausgebildet. Üblicherweise befinden sich mindestens eine Öffnung (Kanülenöffnung) im Bereich der Kanülenspitze und eine weitere Öffnung im Bereich des Kanülenansatzes, so dass eine Kanüle flüssigkeitsdurchströmbar ist. Es gibt aber auch Kanülen mit mehreren Kanülenöffnungen im Bereich der Kanülenspitze oder des Kanülenschaftes. Manche Kanülen weisen an der Kanülenspitze keine Kanülenöffnung auf, sondern nur im Bereich des Kanülenschaftes.

Der Hohlraum im Inneren einer Kanüle wird auch Kanüleninnenraum oder Lumen genannt. Der Kanüleninnenraum reicht vom Kanülenansatz bis zur Kanülenöffnung an der Kanülenspitze und verläuft in der Regel längs durch die gesamte Kanüle. Bei Kanülen ohne Kanülenöffnung an der Kanülenspitze ist ein Teil des Kanüleninnenraumes ein Totraum, nämlich der Abschnitt zwischen der Kanülenöffnung im Bereich des Kanülenschaftes und dem Ende der Kanülenspitze.

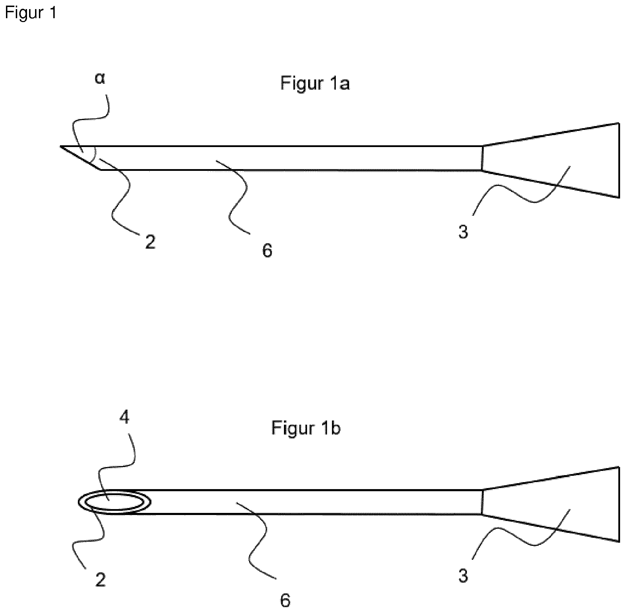

Die Kanülenspitze kann einen schrägen, angeschärften Schliff aufweisen, es handelt sich dann also um eine scharfe Kanüle. Die Kanülen mit einer scharfen Spitze werden für die Punktion verwendet, weil sie das zu durchdringende Gewebe zerschneiden oder verdrängen können. Die Spitze einer Kanüle kann durch den Winkel α beschrieben werden. Der Winkel α ist der Winkel zwischen der Außenseite des Kanülenschaftes, also der Kanülenwand und der abgeschrägten Fläche der Kanülenspitze (Figur 1a).

Die Kanülenspitze kann aber auch stumpf sein. Stumpfe Kanülen haben üblicherweise eine senkrecht angeschnittene Spitze, welche stumpf geschliffen ist. Kanülen mit einer senkrecht angeschnittenen Spitze weisen somit eine Kanülenspitze mit einem Winkel α von 90° auf. Eine besondere Form einer stumpfen Kanüle ist die Knopfkanüle, diese besitzt eine wulstförmig verdickte Spitze. Stumpfe Kanülen werden nicht für eine Punktion verwendet, sondern um einen Zugang zu einer bereits bestehenden Körperöffnung oder Körperhöhle zu legen, beispielsweise um eine Wunde zu spülen. Daher sind stumpfe Kanülen nicht zur Lösung der erfindungsgemäßen Aufgabe geeignet.

Es gibt eine Vielzahl von verschiedenen Schliffformen für eine scharfe Kanülenspitze. Je nachdem wie die geometrische Form der Kanülenspitze ausgebildet ist und wie der Schliff der Kanülenspitze durchgeführt wird, wird das punktierte Gewebe mehr oder weniger bei der Punktion verletzt.

Der Außendurchmesser einer Kanüle wird üblicherweise in Gauge (G) angegeben. Beispielsweise entspricht 27 G dabei einem Außendurchmesser von 0,4 mm, 31 G entspricht dabei einem Außendurchmesser von 0,28 mm. Je höher also der Gauge-Wert ist, desto niedriger ist der Außendurchmesser. Bei Kanülen mit einem hohen Gauge-Wert ist das Ausmaß der Gewebsverletzung bei einer Punktion in der Regel geringer als bei Kanülen mit einem niedrigen Gauge-Wert. Daher sind für die Punktion von sehr empfindlichen Geweben wie z.B. der Glaskörper Kanülen mit einem hohen G-Wert vorteilhaft.

Der G-Wert bestimmt nicht nur den Außendurchmesser einer Kanüle, sondern auch die Stärke der Kanülenwand und somit auch den Innendurchmesser der Kanüle. Beispielsweise weist eine Kanüle mit einem G-Wert von 31 einen Innendurchmesser von 0,12 mm auf und eine Wandstärke von 0,08 mm.

Bei einer Punktion ist in erster Linie die Kanülenspitze für das Ausmaß der Verletzung des punktierten Gewebes maßgeblich, in zweiter Linie der G-Wert der Kanüle. Um also das Ausmaß der Verletzung des punktierten Gewebes möglichst zu reduzieren, können die Kanülen bei diesen beiden Parametern modifiziert werden. Im Allgemeinen ist die Verletzung des punktierten Gewebes bei der Verwendung von geraden Kanülen geringer als bei der Verwendung von gebogenen, gekrümmten oder abgeknickten Kanülen.

Bei der Punktion eines Gewebes werden in Abhängigkeit von der Kanülenform, insbesondere der Form der Kanülenspitze mehr oder weniger viele Gewebeteile von der Außenfläche des Körpers in das Innere des Körpers verschleppt. Diese Gewebeverschleppung ist sehr nachteilig: Erstens, weil durch das verschleppte Gewebe auch Bakterien und andere Mikroorganismen in das Körperinnere verschleppt werden und dort eine Infektion verursachen. Zweitens, weil das verschleppte Gewebe im Körperinneren als Fremdkörper wirkt, vor allem, wenn die verschleppten Gewebezellen im Körperinneren weiter proliferieren und wachsen.

Für bestimmte medizinische Zwecke ist es notwendig, den Glaskörper (*corpus vitreum*) eines menschlichen oder tierischen Auges mit einer Kanüle zu punktieren, beispielsweise um ein Fluid in das Auge oder in den Glaskörper zu injizieren. Bei der Punktion des Glaskörpers müssen die allgemeinen Kautelen für die Punktion des menschlichen oder tierischen Körpers eingehalten werden. Bei der Punktion des Glaskörpers ist die Gewebeverschleppung besonders relevant: Erstens, weil das Augenwandgewebe (z.B. Hornhautgewebe) auf Grund seiner Empfindlichkeit schlecht desinfiziert werden kann und zweitens, weil das verschleppte Augenwandgewebe im Glaskörper verbleibt, dort als Fremdkörper wirkt und sogar proliferieren und wachsen kann und sich zu einer Membran entwickeln kann.

Auf Grund der anatomischen Struktur und Größe des Glaskörpers werden Fluide wie beispielsweise Medikamente in vergleichsweise geringer Menge (also geringe Volumina von Fluiden) in den Glaskörper eingebracht. Daher ist die genaue Dosierung bei der Applikation von Fluiden in den Glaskörper besonders wichtig, um trotz der geringen Fluidmenge den erwünschten medizinischen Effekt zu erreichen.

Auf Grund seiner anatomischen Besonderheiten ist der Glaskörper besonders anfällig gegenüber einer Entzündung (Vitritis, Endophthalmitis), egal ob durch Bakterien oder Mikroorganismen verursacht oder durch Fremdkörper. Eine Endophthalmitis ist eine sehr schwerwiegende Augenerkrankung und führt häufig zum kompletten Verlust eines Auges. Eine Endophthalmitis kann beispielsweise durch das Eindringen eines Fremdkörpers in den Glaskörper (z.B. ein Splitter) verursacht werden oder auch durch das Verschleppen von Hornhautgewebe oder anderes Augenwandgewebe in den Glaskörper. Häufig tritt eine Endophthalmitis auch als Komplikation einer Augenoperation auf, beispielsweise einer Kataraktoperation.

Die Zellen des Augenwandgewebes können nach einer Verschleppung in den Glaskörper in Folge einer Punktion dort weiter proliferieren und z.B. zu einer epiretinalen Membranformation führen. Das ist sehr nachteilig, weil epiretinale Membranen zu Sehstörungen und zu einer Netzhautablösung führen können. Außerdem ist für die Entfernung einer epiretinalen Membran eine aufwendige und komplikationsanfällige Augenoperation nötig.

### Stand der Technik

Für die Punktion des Glaskörpers eines Auges gibt es derzeit keine speziell dafür entwickelten und zugelassenen Kanülen. Alle Kanülen, die derzeit für die Punktion des Glaskörpers verwendet werden, wurden für andere Einsatzzwecke entwickelt.

Folgende Kanülen aus dem Stand der Technik können für die Punktion des Glaskörpers verwendet werden:
**Kanüle mit einer schräg angeschliffenen Spitze:** Es handelt sich dabei um die üblicherweise in der Medizin für eine Punktion verwendete Kanüle, welche beispielsweise zur Punktion einer Vene verwendet wird. Diese Kanülen werden in unterschiedlichen Größen angeboten, beispielsweise von 10 bis 30 Gauge (G). Derzeit werden keine Kanülen mit geringerem Durchmesser (beispielsweise mit 31 G) kommerziell angeboten. Durch den schrägen Schliff der Kanülenspitze weist diese einen sehr scharfen Rand mit einer umlaufenden Schneidkante auf. Dieser scharfe Rand ist im Allgemeinen erwünscht und gewollt, weil dadurch bei der Punktion in das Gewebe ein kleiner Schnitt gesetzt wird. Würde das Gewebe nicht zerschnitten werden sondern nur verdrängt, dann wäre die Punktion für den Patienten deutlich schmerzhafter.

Bei den handelsüblichen Kanülen mit schräg angeschliffener Spitze liegt die Öffnung der Kanüle direkt an der Kanülenspitze und weist durch den schrägen Schliff einen elliptischen Querschnitt auf (Figur 1b). Die Spitzen dieser handelsüblichen Kanülen weisen üblicherweise einen Winkel α von ca. 30-40° auf (Figur 1a). Der Winkel α wird definiert durch die Kanülenwand und die schräge Fläche der Kanülenspitze. Der Winkel α ist ein bestimmender Faktor für die Stabilität der Kanülenspitze. Wenn der Winkel α verringert wird, dann verringert sich auch die Stabilität der Kanülenspitze und diese kann sich bei der Punktion verbiegen, v.a. wenn ein straffes oder hartes Gewebe punktiert wird. Da der Winkel α der Kanülenspitze einen Einfluss auf die Schmerzhaftigkeit bei der Punktion hat (je größer der Winkel α desto schmerzhafter), ist der relativ große Winkel bei diesen Kanülen für die Punktion eines Glaskörpers nachteilig.

Diese Kanülen weisen zusätzlich den wesentlichen Nachteil auf, dass auf Grund der sehr scharfen Ränder der Kanülenspitze bei der Punktion Gewebe ausgestanzt wird, welches sich dann im Kanüleninnenraum befindet. Dieses ausgestanzte Gewebe wird dann bei der Applikation eines Fluids in das punktierte Gewebe gespült und verbleibt dort. Bei der Punktion des Glaskörpers wird also durch diese Kanülen Augenwandgewebe wie z.B. Hornhautgewebe in das Innere des Glaskörpers verschleppt. Dort verursacht das Augenwandgewebe eine Endophthalmitis, welche eine schwerwiegende Komplikation darstellt und zum Verlust des Auges führen kann.

**Kanüle mit einer Kanülenöffnung seitlich an der Kanülenwand:** Bei dieser Kanüle hat die Kanülenspitze keine Kanülenöffnung, sondern ist geschlossen. Die Kanüle weist eine Kanülenöffnung seitlich an der Kanülenwand (Kanülenschaft) auf. Der Kanüleninnenraum reicht bis zur Kanülenspitze, wodurch zwischen der Kanülenöffnung und der Kanülenspitze ein Totraum entsteht. Dieser Totraum in der Kanülenspitze ist aus folgenden Gründen nachteilig: In ihm sammelt sich ein Teil des zu applizierenden Fluids an und kann nicht für den Patienten genutzt werden. Da das Volumen des Totraumes unbekannt ist, wird dadurch die Genauigkeit der Dosierung des applizierten Fluids verringert. Da die Kanüle nach dem Gebrauch entsorgt wird, wird gleichzeitig auch der sich im Totraum befindliche Fluidrest verworfen, was eine Verschwendung von Fluid darstellt. Zusätzlich ist durch den Totraum in der hohlen Kanülenspitze die Stabilität der Kanülenspitze eingeschränkt, was bei der Punktion des verhältnismäßig straffen und zähen Augenwandgewebes ein Problem ist. Bei unsachgemäßer Handhabung kann sich die hohle Kanülenspitze verbiegen oder sogar abbrechen.

Die Kanülenspitze weist einen Winkel α von ca. 30-40° auf. Dieser Winkel ist vergleichbar mit dem Winkel bei den bekannten Kanülen mit einer schräg angeschliffenen Spitze. Der relativ große Winkel bei dieser Kanüle ist für die Punktion eines Glaskörpers nachteilig, weil dadurch die Schmerzen bei der Punktion für den Patienten größer sind als bei einer Kanüle mit einem kleineren Winkel α.

**Spinalkanüle:** Spinalkanülen sind für die Punktion des äußeren Liquorraumes im Bereich der Arachnoidea oder des Subarachnoidalraumes (z. B. im Bereich des Lumbalraumes) vorgesehen. Sie müssen eine ausreichende Formstabilität aufweisen, damit das straffe Gewebe der Hirn- und Rückenmarkshäute durchschnitten werden kann. Daher weisen sie bauliche Besonderheiten im Vergleich zu handelsüblichen Kanülen mit einer schräg angeschnittenen Spitze auf: Entweder ist die Kanülenwand stärker dimensioniert und damit formstabiler oder es wird ein Mandrin (Führungsnadel) verwendet, der den Kanüleninnenraum während der Punktion verschließt. Ein Mandrin verhindert zusätzlich auch, dass durch die Spinalkanüle Hautpartikel in den Lumbal- bzw. Arachnoidalraum verschleppt werden.

Es gibt verschiedene Arten von Spinalkanülen, die meist nach ihren Erfindern benannt wurden, beispielsweise die Sprotte-Kanüle, die Quincke-Kanüle oder die Whitacre-Kanüle. Die kommerziell erhältlichen Spinalkanülen haben G-Werte von kleiner als 20 G, das heißt, Spinalkanülen weisen einen verhältnismäßig großen Durchmesser auf und sind daher nicht für die Punktion eines Glaskörpers geeignet.

Spinalkanülen können eine schräg angeschliffene Kanülenspitze mit einer Kanülenöffnung aufweisen (z.B. die Quincke-Kanüle), oder eine kegelförmige Kanülenspitze mit einem Punktschliff oder Pencil-Point-Schliff (z.B. die Sprotte-Kanüle oder die Kanüle nach Whitacre) oder eine senkrecht angeschliffene Kanülenspitze, welche durch einen Mandrin verschlossen wird (z.B. die Ball-Point-Kanüle oder Ball-Pen-Kanüle). Bei Kanülen mit einem Pencil-Point-Schliff befindet sich die Öffnung der Kanüle üblicherweise nicht direkt an der Kanülenspitze, sondern in Richtung des Kanülenansatzes verschoben seitlich an der Kanülenwand im Bereich des Kanülenschafts. Bei den bekannten Kanülen mit einer Kanülenöffnung seitlich an der Kanülenwand reicht der Kanüleninnenraum bis zum Ende der Kanülenspitze. Es entsteht dadurch ein Totraum in der Kanülenspitze im Abschnitt zwischen der seitlichen Kanülenöffnung und dem spitzen Ende der Kanülenspitze.

Dieser Totraum in der Kanülenspitze ist aus folgenden Gründen nachteilig: In ihm sammelt sich ein Teil des zu applizierenden Fluids an und kann nicht für den Patienten genutzt werden. Da das Volumen des Totraumes unbekannt ist, wird dadurch die Genauigkeit der Dosierung des applizierten Fluids verringert. Da die Kanüle nach dem Gebrauch entsorgt wird, wird gleichzeitig auch der sich im Totraum befindliche Fluidrest verworfen, was eine Verschwendung von Fluid darstellt. Durch den Totraum ist die Stabilität der Kanülenspitze eingeschränkt, was bei der Punktion des verhältnismäßig straffen und zähen Augenwandgewebes ein Problem ist. Bei unsachgemäßer Handhabung kann sich die Kanülenspitze verbiegen oder sogar abbrechen.

Die Quincke-Kanüle weist durch den schrägen Schliff an der Kanülenspitze einen sehr scharfen Rand auf. Das hat den Nachteil, dass auf Grund ihrer schräg angeschliffenen Kanülenspitze und auf Grund des fehlenden Mandrins Gewebe bei der Punktion ausgestanzt wird und in das Körperinnere bis hin zum Liquorraum verschleppt wird. Auf Grund des fehlenden Mandrins muss die Quincke-Kanüle eine verhältnismäßig stark dimensionierte Kanülenwand aufweisen, somit sind Quincke-Kanülen mit hohem G-Wert nicht erhältlich. Quincke-Kanülen stanzen also große Gewebestücke bei der Punktion aus und verschleppen diese in den Glaskörper. Außerdem verursachen sie auf Grund ihres kleinen G-Werts bei der Punktion deutlich stärkere Schmerzen für den Patienten im Vergleich zu Kanülen mit einem hohen G-Wert.

Die Spinalkanülen mit einem Pencil-Point-Schliff (z.B. Sprotte-Kanüle oder Kanüle nach Whitacre) haben den Nachteil, dass durch den Pencil-Point-Schliff der Kanülenspitze das durchdrungene Gewebe während der Punktion zur Seite gedrängt wird, was für den Patienten sehr schmerzhaft ist. Durch den Totraum in der Kanülenspitze zwischen der seitlichen Kanülenöffnung und der Kanülenspitze ist die Dosiergenauigkeit bei der Verabreichung von Fluiden sehr eingeschränkt. Außerdem verursacht dieser Totraum eine Fluidverschwendung. Außerdem verursachen sie auf Grund ihres kleinen G-Werts bei der Punktion deutlich stärkere Schmerzen für den Patienten im Vergleich zu Kanülen mit einem hohen G-Wert.

Die Spinalkanülen mit einer senkrecht angeschliffenen Kanülenspitze (z.B. Ball-Point-Kanüle oder Ball-Pen-Kanüle) müssen einen Mandrin aufweisen, damit eine Punktion überhaupt möglich ist. Der Mandrin ist ein zusätzlicher Kostenfaktor, der bei einem Einmal-Wegwerfprodukt, wie es die Kanüle darstellt, signifikant die Herstellungskosten erhöht. Außerdem erschwert der Mandrin die Handhabung der Kanüle bei der Punktion, denn ein Mandrin kann sich verbiegen oder verschieben und erfüllt dann nicht mehr seinen vorgesehenen Zweck. Außerdem verursachen sie auf Grund ihres kleinen G-Werts bei der Punktion deutlich stärkere Schmerzen für den Patienten im Vergleich zu Kanülen mit einem hohen G-Wert.

**Flügelkanüle (Butterfly, Schmetterlingkanüle):** Flügelkanülen sind dadurch gekennzeichnet, dass es sich um meist sehr dünne Kanülen handelt (sie weisen also einen hohen G-Wert auf) und mit zwei biegsamen Flügel aus Kunststoff und einem Schlauch versehen sind. Der Schlauch hat die Funktion eines Ansatzstückes, da er an seinem Ende mit einer Spritze verbindbar ist, beispielsweise mittels eines Luer-Lock-Anschlusses.

Die beiden biegsamen Flügel sind so an der Kanüle befestigt, dass sie zusammen geklappt werden können und somit einen Griff zum Halten der Kanüle während der Punktion bilden. Damit kann die Flügelkanüle sicher und präzise während der Punktion geführt werden.

Die Spitzen der bekannten Flügelkanülen sind schräg angeschliffen und weisen damit einen sehr scharfen Rand auf. Der Winkel α der Kanülenspitze beträgt 30 bis 40°. Die Kanülenöffnung ist direkt an der Kanülenspitze. Die Flügelkanülen weisen daher eine baugleiche Spitze auf wie die herkömmlichen Kanülen mit schräg angeschliffener Spitze und ohne Flügel. Daher werden auch bei Flügelkanülen während der Punktion Gewebestücke ausgestanzt und in das Körperinnere verschleppt.

Zusammengefasst kann festgestellt werden, dass die Kanülen aus dem Stand der Technik entweder einen zu großen Außendurchmesser (also zu kleinen G-Wert) aufweisen, oder Gewebestücke ausstanzen und in das Körperinnere verschleppen, oder zu teuer in der Herstellung sind, oder eine Verschwendung von Fluiden verursachen, oder keine genaue Dosierung von Fluiden ermöglichen oder zu schmerzhaft für den Patienten bei der Punktion sind.

### Aufgabe

Die Aufgabe der vorliegenden Erfindung ist es, eine Kanüle für die Punktion des Glaskörpers eines Auges zur Verfügung zu stellen, durch welche kein Gewebe in das Augeninnere verschleppt wird. Gleichzeitig soll die erfindungsgemäße Kanüle eine möglichst schmerzarme Punktion ermöglichen. Die erfindungsgemäße Kanüle soll eine stabile Kanülenspitze aufweisen. Außerdem soll durch die erfindungsgemäße Kanüle eine genaue Dosierung des applizierten Fluides selbst bei sehr kleinen Volumina ermöglicht werden und es soll kein Fluid verschwendet werden.

### Lösung der Aufgabe

Die erfindungsgemäße Aufgabe wird gelöst durch die Vorrichtung aus Anspruch 1. Die Vorrichtung aus Anspruch 1 ist eine Kanüle 1 mit einer besonderen Geometrie der Kanülenspitze 2 und der mindestens einen Kanülenöffnung 4, so dass bei der Punktion mit der erfindungsgemäßen Kanüle kein Gewebe in das Innere des Körpers verschleppt wird und eine schmerzarme Punktion durchgeführt wird.

Die erfindungsgemäße Kanüle 1 lässt sich in drei Abschnitte gliedern, nämlich in die Kanülenspitze 2 (Abschnitt 1), in den Kanülenansatz 3 (Abschnitt 3) und den dazwischenliegenden Kanülenschaft 6 (Abschnitt 2). Der Abschnitt 1 reicht vom spitzen Ende der Kanülenspitze bis zum Rand der mindestens einen Kanülenöffnung. Dieser Abschnitt ist nicht hohl sondern massiv, die Kanülenspitze weist also keinen Kanüleninnenraum auf. Der Abschnitt 2 reicht vom Rand der mindestens einen Kanülenöffnung bis zum Abschnitt 3. Der Abschnitt 3 entspricht dem Kanülenansatz 3. Die erfindungsgemäße Kanüle ist so ausgeführt, dass ein Fluid vom Kanülenansatz 3 durch den Kanülenschaft bis zur mindestens einen Kanülenöffnung 4 am Ende des Kanülenschaftes, welcher der Kanülenspitze zugewandt ist, fließen kann. Die erfindungsgemäße Kanüle ist also flüssigkeitsdurchströmbar ausgebildet von dem Kanülenansatz 3 durch den Kanülenschaft bis zu der mindestens einen Kanülenöffnung 4.

Die Kanülenspitze 2 (Abschnitt 1) ist geeignet für die Punktion eines menschlichen oder tierischen Auges ausgeführt. Die Kanülenspitze 2 ist mit dem Kanülenschaft (Abschnitt 2) einstückig ausgeführt. Die erfindungsgemäße Kanülenspitze 2 ist nicht hohl, sondern massiv ausgeführt. Die Kanülenspitze 2 weist also keinen Kanüleninnenraum 5 auf.

Der Kanülenschaft (Abschnitt 2) ist hohl und somit flüssigkeitsdurchströmbar. Er ist der Abschnitt der erfindungsgemäßen Kanüle zwischen dem Kanülenansatz 3 (Abschnitt 3) und der mindestens einen Kanülenöffnung 4.

Der Kanülenansatz 3 (Abschnitt 3) ist hohl und somit flüssigkeitsdurchströmbar. Der Kanülenansatz 3 kann mit dem Kanülenschaft (Abschnitt 2) und der Kanülenspitze (Abschnitt 1) einstückig ausgeführt sein. Alternativ kann der Kanülenansatz 3 als eigenes Bauteil ausgeführt sein, welches mit dem Kanülenschaft (Abschnitt 2) verbindbar ausgeführt ist. Der Kanülenansatz 3 ist so ausgebildet, dass eine Spritze oder ein Schlauch oder ein Reservoir angeschlossen werden kann, aus dem das zu verabreichende Fluid in den Glaskörper appliziert werden kann.

Die erfindungsgemäße Kanüle 1 weist die folgenden Merkmale auf:
- Der Kanüleninnenraum 5 reicht nicht bis in die Kanülenspitze 2, sondern endet vor der Kanülenspitze. Die Kanülenspitze weist also keine Öffnung auf, sondern ist geschlossen.
- Die Kanülenspitze ist nicht hohl sondern massiv ausgebildet. Dadurch weist die Kanülenspitze keinen Totraum auf.
- Die Kanülenspitze ist scharf angeschliffen, ihre Schnittlinien sind scharfe Schneidekanten.
- Die Kanülenöffnung 4 liegt von der Kanülenspitze entfernt in Richtung zum Kanülenansatz 3 an der Seite der Kanüle in der Kanülenwand 6 im Bereich des Kanülenschaftes.
- Der Rand der Kanülenöffnung 4 ist abgerundet. Diese Abrundung kann konvex ausgeführt sein (beispielsweise als Halbkreis oder Viertelkreis) oder konkav (beispielsweise als Hohlkehle).
- Der Rand der Kanülenöffnung 4 kann zusätzlich poliert sein. Dadurch wird eine besonders glatte Oberfläche im Bereich der Kanülenöffnung 4 erreicht.
- Die Kanülenöffnung 4 kann rund, oval oder schlitzförmig sein.
- Die Kanüle kann alternativ zwei oder mehr Kanülenöffnungen 4 aufweisen.
- Die Kanüle weist ein Kaliber zwischen 27 und 33 G auf.
- Die Kanülenspitze weist einen Winkel α von weniger als 30° auf, bevorzugt unter 20°, beispielsweise ca. 18°. Dadurch ist die Spitze der erfindungsgemäßen Kanüle deutlich spitzer als bei den Kanülen im Stand der Technik.

Die fehlende Kanülenöffnung 4 an der Kanülenspitze 2 bewirkt, dass bei der Punktion keine Gewebsstanze durch die Kanülenöffnung 4 ausgestochen werden kann. Die fehlende Kanülenöffnung 4 an der Kanülenspitze 2 macht einen Mandrin überflüssig. Der abgerundete Rand der Kanülenöffnung 4 bewirkt ebenfalls, dass bei der Punktion keine Gewebsstanze ausgestochen wird. Durch die zusätzliche Polierung des Randes der Kanülenöffnung 4 wird der Grad der Schädigung des punktierten Gewebes während der Punktion weiter verringert.

Die scharf angeschliffene Kanülenspitze bewirkt, dass bei der Punktion das zu durchdringende Gewebe geschnitten wird und nicht nur verdrängt. Dadurch werden die Schmerzen bei der Punktion für den Patienten verringert. Die schräge Fläche der erfindungsgemäßen Kanülenspitze kann gerade, konvex oder konkav ausgeführt werden.

Der sehr geringe Durchmesser der erfindungsgemäßen Kanüle zwischen 27 und 33 G bewirkt, dass sowohl der Schmerz bei der Punktion für den Patienten als auch der Grad der Gewebsschädigung bei der Punktion verringert wird. Der mögliche Nachteil des hohen G-Wertes auf die Stabilität der Kanüle, vor allem auf die Stabilität der Kanülenspitze, wird dadurch ausgeschlossen, dass die erfindungsgemäße Kanülenspitze nicht hohl sondern massiv ausgeführt ist.

Der kleine Winkel α der Kanülenspitze 2 von weniger als 30°, bevorzugt unter 20°, beispielsweise ca. 18° bewirkt, dass das Punktieren des Augengewebes mit weniger Druck durchführbar ist. Dadurch ermöglicht der kleine Winkel der Kanülenspitze 2 also eine deutlich schonendere Punktion. Ebenso bewirkt der kleine Winkel der Kanülenspitze 2 von weniger als 30°, bevorzugt weniger als 20°, beispielsweise ca. 18°, dass das punktierte Gewebe weniger geschädigt wird. Dieser geringe Winkel α der Kanülenspitze ist möglich, weil die Kanülenspitze nicht hohl ist sondern massiv.

Die erfindungsgemäße Ausgestaltung der Kanülenspitze 2 bewirkt, dass der Kanüleninnenraum 5 keine Toträume aufweist, da die Kanülenspitze 2 nicht hohl, sondern massiv ist. Zusätzlich wird dadurch die mechanische Stabilität der Kanüle erhöht, sodass selbst dünne Kanülen mit einem hohen G-Wert zwischen 27 und 33 eine gute Stabilität bei der Punktion aufweisen, ohne dass ein Mandrin nötig ist. Der fehlende Totraum hat auch den Vorteil, dass bei der Applikation eines Fluids dieses nicht im Totraum verloren geht. Somit ist die Dosiergenauigkeit besser als bei den Kanülen aus dem Stand der Technik mit einer seitlichen Kanülenöffnung und einem Totraum in der Kanülenspitze.

Der Kanülenansatz 3 kann gemäß des Luer-Systems als Luer-Lock- oder Luer-Slip-Anschluss ausgestaltet sein. Der Kanülenansatz 3 kann alternativ gemäß des Rekord-Spritzensystems ausgestaltet sein. Der Kanülenansatz 3 kann alternativ gemäß des Zylinderampullen-Spritzensystems ausgestaltet sein. Der Kanülenansatz 3 kann alternativ auch ein Schlauch sein, der mit einer Spritze verbindbar ausgestaltet ist.

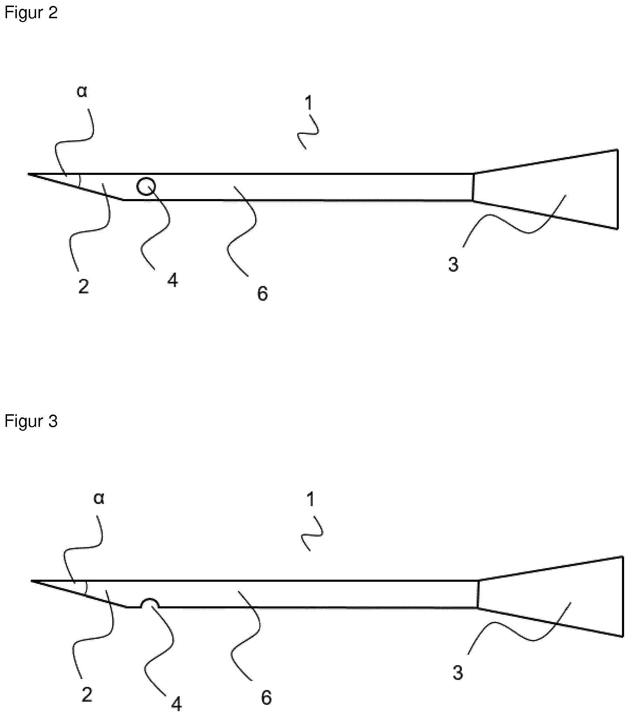

Vorteilhaft liegt die mindestens eine Kanülenöffnung 4 ca. 1 mm von der Kanülenspitze 2 entfernt an der Kanüle 1 (Figur 2 und 3). Mit dieser Anordnung der Kanülenöffnung ist eine intravitrealen Injektion am besten durchführbar. Wenn mehr als eine Kanülenöffnung 4 vorgesehen sind, dann können die Kanülenöffnungen rund um den Kanülenschaft herum verteilt angeordnet sein. Alternativ können die Kanülenöffnungen in einer Reihe entlang des Kanülenschaftes angeordnet sein.

In einer alternativen Ausführungsform kann die erfindungsgemäße Kanüle als Flügelkanüle (Schmetterlingkanüle, Butterfly-Kanüle) ausgebildet sein. Sie weist also zusätzlich zwei biegsame Flügel und einen Schlauch auf. In diesem Fall ist der Schlauch als Kanülenansatz 3 ausgebildet. Alternativ werden nur die zwei biegsamen Flügel angebracht, so dass ein Kanülenansatz 3 gemäß des Standes der Technik verwendet wird.

Die erfindungsgemäße Kanüle 1 weist vorzugsweise eine Kanülenöffnung 4 auf. In einem Ausführungsbeispiel kann die Kanüle 1 auch zwei oder mehr Kanülenöffnungen 4 aufweisen, die verteilt am Kanülenschaft angeordnet sind. Unabhängig von den ein oder mehr Kanülenöffnungen 4 weist die erfindungsgemäße Kanüle eine weitere Öffnung des Kanülenschaftes am Kanülenansatz 3 auf, damit die Kanüle 1 flüssigkeitsdurchströmbar ist. Die Ausführungsform mit 2 oder mehr Kanülenöffnungen 4 hat den Vorteil, dass bei der Applikation eines Fluids in den Glaskörper dieses Fluid gleichmäßiger im Glaskörper verteilt wird.

Die erfindungsgemäße Kanüle kann aus Stahl hergestellt werden, der Kanülenansatz 3 sowie die Flügel und der Schlauch der Butterflykanüle aus einem geeigneten Kunststoff. Es können aber auch andere geeignete Materialien für die Produktion der erfindungsgemäßen Kanüle verwendet werden.

### Abbildungslegende

**Figur 1** zeigt eine Kanüle gemäß des Standes der Technik mit schräg angeschliffener Spitze in einer seitlichen Ansicht (Figur 1a) und in einer Aufsicht (Figur 1b). Die Kanüle weist an einem Ende einen Kanülenansatz 3 auf und an dem anderen Ende eine Kanülenspitze 2. Die Kanülenöffnung 4 befindet sich direkt an der Kanülenspitze 2. Der Winkel α der Kanülenspitze wird gebildet durch die Kanülenwand und die schräge Fläche der Kanülenspitze. Der Winkel α der Kanülenspitze beträgt ca. 30-40°. Der Kanüleninnenraum 5 ist bei dieser Darstellung nicht zu sehen. Er befindet sich im Inneren der Kanüle und reicht von dem Kanülenansatz 3 durch den Kanülenschaft bis zur Kanülenöffnung 4 an der Kanülenspitze 2.
**Figur 2** zeigt eine seitliche Ansicht auf die erfindungsgemäße Kanüle 1 mit der Kanülenspitze 2, dem Kanülenansatz 3 und einer Kanülenöffnung 4. Die Kanülenöffnung 4 befindet sich nicht direkt an der Kanülenspitze 2, sondern ca. 1 mm davon entfernt in Richtung des Kanülenansatzes 3 seitlich an der Kanüle im Bereich des Kanülenschaftes 6. In diesem Ausführungsbeispiel ist die Kanülenöffnung 4 an der seitlichen Fläche des Kanülenschaftes 6 angeordnet. Der Winkel α der Kanülenspitze wird gebildet durch die Kanülenwand und die schräge Fläche der Kanülenspitze. Der Winkel α der Kanülenspitze ist kleiner als 30°. Der Kanüleninnenraum 5 ist bei dieser Darstellung nicht zu sehen, er befindet sich im Inneren der Kanüle.
**Figur 3** zeigt eine seitliche Ansicht auf die erfindungsgemäße Kanüle 1 mit der Kanülenspitze 2, dem Kanülenansatz 3 und einer Kanülenöffnung 4. Die Kanülenöffnung 4 befindet sich nicht direkt an der Kanülenspitze 2, sondern ca. 1 mm davon entfernt in Richtung des Kanülenansatzes 3 seitlich an der Kanüle im Bereich des Kanülenschaftes 6. In diesem bevorzugten Ausführungsbeispiel ist die Kanülenöffnung 4 an jener Fläche des Kanülenschaftes 6 angeordnet, von der auch die Kanülenspitze angeschrägt ist. Der Winkel α der Kanülenspitze ist kleiner als 30°. Der Kanüleninnenraum 5 ist bei dieser Darstellung nicht zu sehen, er befindet sich im Inneren der Kanüle.

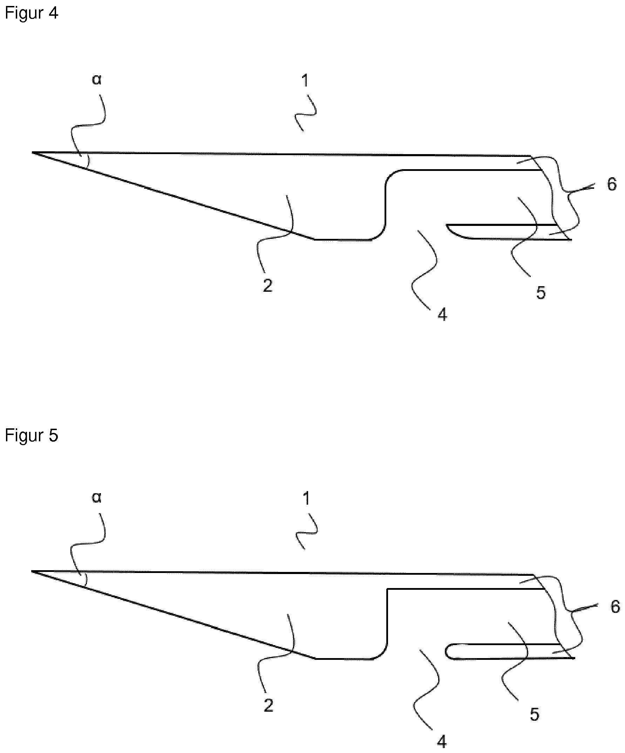

**Figur 4** zeigt einen Längsschnitt durch die Abschnitte 1 und 2 der erfindungsgemäßen Kanüle 1 auf der Höhe einer Kanülenöffnung 4 mit der Kanülenspitze 2 und der Kanülenöffnung 4. Der Kanülenansatz 3 ist nicht dargestellt. Die Kanülenöffnung 4 befindet sich nicht direkt an der Kanülenspitze 2, sondern ca. 1 mm davon entfernt an dem Kanülenschaft 6. Der Rand der Kanülenöffnung 4 ist abgerundet. Der Winkel α der Kanülenspitze ist kleiner als 30°. Der Kanüleninnenraum 5 endet an der Kanülenöffnung 4 und reicht nicht in die Kanülenspitze 2. Der Rand der Kanülenöffnung ist konvex abgerundet.
**Figur 5** zeigt ein weiteres Ausführungsbeispiel der erfindungsgemäßen Kanüle 1. Sie zeigt einen Längsschnitt durch die Abschnitte 1 und 2 der erfindungsgemäßen Kanüle 1 auf der Höhe einer Kanülenöffnung 4 mit der Kanülenspitze 2 und der Kanülenöffnung 4. Der Kanülenansatz 3 ist nicht dargestellt. Die Kanülenöffnung 4 befindet sich nicht direkt an der Kanülenspitze 2, sondern ca. 1 mm davon entfernt an dem Kanülenschaft 6. Der Winkel α der Kanülenspitze ist kleiner als 30°. Der Kanüleninnenraum 5 endet an der Kanülenöffnung 4 und reicht nicht in die Kanülenspitze 2. Der Rand der Kanülenöffnung 4 ist konvex abgerundet, wobei diese Abrundung bis in den Kanüleninnenraum 5 reicht.

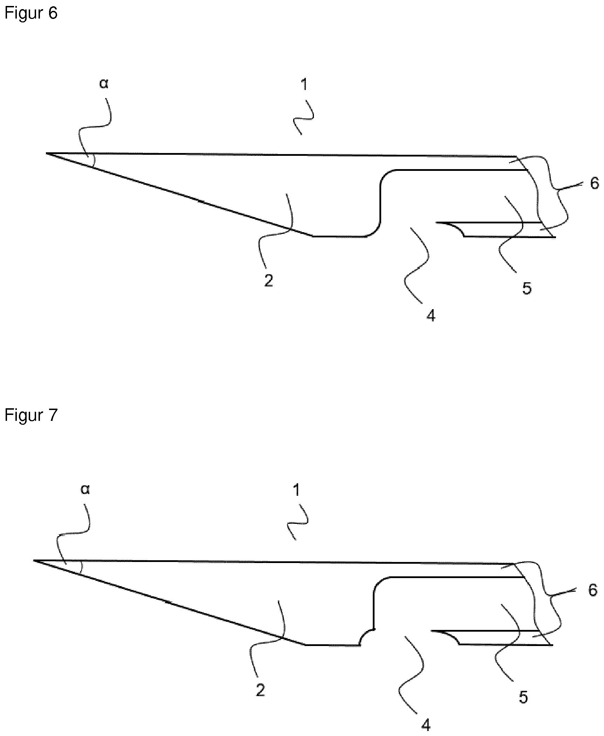

**Figur 6** zeigt ein weiteres Ausführungsbeispiel der erfindungsgemäßen Kanüle 1. Sie zeigt einen Längsschnitt durch die Abschnitte 1 und 2 der erfindungsgemäßen Kanüle 1 auf der Höhe einer Kanülenöffnung 4 mit der Kanülenspitze 2 und der Kanülenöffnung 4. Der Kanülenansatz 3 ist nicht dargestellt. Die Kanülenöffnung 4 befindet sich nicht direkt an der Kanülenspitze 2, sondern ca. 1 mm davon entfernt an dem Kanülenschaft 6. Der Winkel α der Kanülenspitze ist kleiner als 30°. Der Kanüleninnenraum 5 endet an der Kanülenöffnung 4 und reicht nicht in die Kanülenspitze 2. Der Rand der Kanülenöffnung 4 ist teils konvex, teils konkav abgerundet.
**Figur 7** zeigt ein weiteres Ausführungsbeispiel der erfindungsgemäßen Kanüle 1. Sie zeigt einen Längsschnitt durch die Abschnitte 1 und 2 der erfindungsgemäßen Kanüle 1 auf der Höhe einer Kanülenöffnung 4 mit der Kanülenspitze 2 und der Kanülenöffnung 4. Der Kanülenansatz 3 ist nicht dargestellt. Die Kanülenöffnung 4 befindet sich nicht direkt an der Kanülenspitze 2, sondern ca. 1 mm davon entfernt an dem Kanülenschaft 6. Der Winkel α der Kanülenspitze ist kleiner als 30°. Der Kanüleninnenraum 5 endet an der Kanülenöffnung 4 und reicht nicht in die Kanülenspitze 2. Der Rand der Kanülenöffnung 4 ist konkav abgerundet.

### Bezugszeichenliste

1 Kanüle zur Punktion des Glaskörpers
2 Kanülenspitze
3 Kanülenansatz
4 Kanülenöffnung
5 Kanüleninnenraum
6 Kanülenschaft

α Winkel der Kanülenspitze zwischen der Kanülenwand und der schrägen Fläche der Kanülenspitze

## Patentansprüche

1. Kanüle (1) zur Punktion des Glaskörpers (*corpus vitreum*) eines menschlichen oder tierischen Auges und zur Injektion eines Fluides in den Glaskörper (*corpus vitreum*) eines menschlichen oder tierischen Auges umfassend einen flüssigkeitsdurchströmbaren Kanülenansatz (3), eine scharf angeschliffene Kanülenspitze (2), einen flüssigkeitsdurchströmbaren Kanülenschaft (6) und mindestens eine abgerundete Kanülenöffnung (4) im Bereich des Kanülenschaftes, **dadurch gekennzeichnet, dass** die Kanülenspitze einen Winkel α von weniger als 30° aufweist und dass die Kanülenspitze massiv ausgebildet ist.

2. Kanüle nach Anspruch 1, **dadurch gekennzeichnet, dass** zwei oder mehr Kanülenöffnungen (4) an dem Kanülenschaft (6) angeordnet sind.

3. Kanüle nach Anspruch 1 oder 2, **dadurch gekennzeichnet, dass** der Winkel α an der Kanülenspitze (2) kleiner als 20° ist.

4. Kanüle nach einem der vorhergehenden Ansprüche, **dadurch gekennzeichnet, dass** die mindestens eine Kanülenöffnung (4) rund, oval oder schlitzförmig ist.

5. Kanüle nach einem der vorhergehenden Ansprüche, **dadurch gekennzeichnet, dass** die Kanüle ein Kaliber zwischen 27 und 33 G aufweist.

6. Kanüle nach einem der vorhergehenden Ansprüche, **dadurch gekennzeichnet, dass** die Kanüle als Flügelkanüle (Schmetterlingkanüle, Butterfly-Kanüle) ausgebildet ist.

7. Verwendung der Kanüle (1) nach Anspruch 1 zur Punktion des Glaskörpers *(corpus vitreum)* eines menschlichen oder tierischen Auges und zur Injektion eines Fluides in den Glaskörper *(corpus vitreum)* eines menschlichen oder tierischen Auges.
